Europäisches Patentamt

(19) European Patent Office

Office européen des brevets

(11) EP 0 729 932 A1

(12) EUROPEAN PATENT APPLICATION

(43) Date of publication:
04.09.1996 Bulletin 1996/36

(51) Int. Cl.⁶: **C07C 17/278**, C07C 17/20, C07C 19/08, C07C 19/01

(21) Application number: 96103220.8

(22) Date of filing: 01.03.1996

(84) Designated Contracting States:
DE FR GB IT

(30) Priority: 03.03.1995 JP 44093/95
03.03.1995 JP 44094/95

(71) Applicant: CENTRAL GLASS COMPANY, LIMITED
Yamaguchi 755 (JP)

(72) Inventors:
• Tamai, Ryoichi
Kawagoe-shi, Saitama 350-11 (JP)

• Yoshikawa, Satoshi
Kawagoe-shi, Saitama 350-11 (JP)
• Sakyu, Fuyuhiko
Kawagoe-shi, Saitama 350-11 (JP)
• Hibino, Yasuo
Kawagoe-shi, Saitama 350-11 (JP)

(74) Representative: Morgan, James G. et al
Robert-Koch-Strasse 1
80538 München (DE)

(54) **Method of producing halopropane**

(57) The invention relates to a first method of producing 1,1,1,3,3-pentachloropropane, and a second method of producing 1,1,1,3,3-pentafluoropropane from 1,1,1,3,3-pentachloropropane. In the first method, carbon tetrachloride is reacted with vinyl chloride in an aprotic polar organic solvent, in the presence of a catalyst containing elemental iron. In the second method, 1,1,1,3,3-pentachloropropane is fluorinated with hydrogen fluoride in a liquid phase, in the presence of an antimony-containing catalyst. According to the first and second methods, 1,1,1,3,3-pentachloropropane and 1,1,1,3,3-pentafluoropropane can be respectively produced with high yield in an industrial scale.

## Description

### BACKGROUND OF THE INVENTION

The present invention relates to a method of producing 1,1,1,3,3-pentachloropropane and another method of producing 1,1,1,3,3-pentafluoropropane from 1,1,1,3,3-pentachloropropane.

Chlorofluorocarbons are considered to be detrimental toward the Earth's ozone layer. Therefore, there has been a world-wide effort to develop alternative substances such as hydrochlorofluorocarbons and hydrofluorocarbons. Of these, 1,1,1,3,3-pentafluoropropane is useful as a foaming agent for foaming substances such as polyolefin, polystyrene, polyurethane and polyisocyanurate, and as a refrigerant for turbo refrigerator.

There have proposed several methods for producing 1,1,1,3,3-pentafluoropropane. For example, JP-A-Hei-6-256235 discloses a method of producing 1,1,1,3,3-pentafluoropropane from $CF_3$-CClX-$CF_2$Cl where X is hydrogen or chlorine, by catalytic hydrogenation. A preferable catalyst for this method is a common hydrogenation catalyst. U.S. Patent No. 2,942,036 discloses a method of hydrogenating 1,2,2-trichloropentafluoropropane to produce 1,1,1,3,3-pentafluoropropane or 1,1,3,3,3-pentafluoro-1-propene or mixtures thereof. A catalyst for this method is palladium supported on activated carbon. These two methods are superior in conversion and selectivity. However, these catalysts deteriorate considerably in these methods. Furthermore, it is necessary to prepare the raw material(s) of these methods in advance. Thus, these methods may not be suitable for the production of 1,1,1,3,3-pentafluoropropane in an industrial scale.

There is disclosed, in published English translation (pp. 1312-1317) of Izvestiya Akademii Nauk SSSR, Otdelenie Khimicheskikh Nauk, No. 8, pp. 1412-1418, August, 1960 (CA 55, 349f), a method of producing 1,1,1,3,3-pentafluoropropane by hydrogenating 1,1,3,3,3-pentafluoro-1-propene in the presence of Pd-$Al_2O_3$. However, it is difficult to find the raw material of this method (i.e., 1,1,3,3,3-pentafluoro-1-propene) on the market.

There are known several methods for synthesizing chloropropanes that is, chlorination of propane, radical addition reactions of chloromethanes with olefins each having a carbon atom number of 2, and ion addition reactions such as Friedel-Crafts reaction of and Prins reaction of olefins each having a carbon atom number of 2 with chloromethanes.

There are provided, for example, the following first, second, third, and fourth methods for producing chloroalkane(s) by reacting olefin(s) with carbon tetrachloride. The first method is characterized in that an organic peroxide is used as a reaction additive (see U.S. Patent No. 2,440,800). The second method is characterized in that a combination of a metal salt of an organic or inorganic acid and an amine(s) and the like is used as a catalyst (see JP-B-Sho-37-18389, JP-B-Sho-39-28306, JP-B-Sho-40-19740 and JP-B-Sho-41-20692). The third method is characterized in that a catalyst composed of metallic copper and an alkali metal halide(s) is used in the reaction (see JP-A-Sho-47-31907). The fourth method is characterized in that a catalyst composed of a polyalkoxide(s) and iron chloride is used in the reaction (see JP-A-Sho-52-59102). The fifth method is characterized in that a catalyst composed of phosphorous alkylester, iron chloride and nitrile compound(s) is used in the reaction(see JP-A-Sho-52-59103).

There are provided, for example, the following first, second, and third methods for producing 1,1,1,3,3-pentachloropropane. In the first method, vinylidene chloride is reacted with chloroform in the presence of copper-amine catalyst (see M. Kotora et al. (1991) React. Kinet. Catal. Lett., Vol. 44, No. 2, pp. 415-419). In the second method, carbon tetrachloride is reacted with vinyl chloride in the presence of copper-amine catalyst (see M. Kotora et al. (1992) J. of Molecular Catalysis, pp. 51-60). In the third method, carbon tetrachloride is reacted with vinyl chloride in an isopropanol solvent, in the presence of a ferrous chloride catalyst (see E. N. Zil'berman et al. (1967) J. of Org. Chem. USSR, Vol. 3, pp. 2101-2105).

### SUMMARY OF THE INVENTION

It is a first object of the present invention to provide a method of producing 1,1,1,3,3-pentachloropropane with high yield, which method is advantageous in an industrial scale production.

It is a second object of the present invention to provide a method of producing 1,1,1,3,3-pentafluoropropane from 1,1,1,3,3-pentachloropropane with high yield, which method is advantageous in an industrial scale production.

According to the present invention, there is provided a first method of producing 1,1,1,3,3-pentachloropropane, comprising a step of reacting carbon tetrachloride with vinyl chloride in an aprotic polar organic solvent, in the presence of a catalyst comprising elemental iron.

According to the present invention, there is provided a second method of producing 1,1,1,3,3-pentafluoropropane, comprising a step of fluorinating 1,1,1,3,3-pentachloropropane with hydrogen fluoride in a liquid phase, in the presence of a catalyst comprising at least one of metallic antimony and an antimony compound.

In order to achieve the first object of the invention, the inventors have eagerly examined telomerizations of vinyl chloride with carbon tetrachloride in the presence of catalyst, and unexpectedly found that 1,1,1,3,3-pentachloropropane is selectively produced with high yield by reacting carbon tetrachloride with vinyl chloride in an aprotic polar organic solvent, in the presence of a catalyst comprising elemental iron.

Furthermore, the inventors have unexpectedly found that 1,1,1,3,3-pentafluoropropane is produced with high yield by fluorinating 1,1,1,3,3-pentachloropropane with hydrogen fluoride in a liquid phase, in the presence of a catalyst comprising at least one of metallic antimony and an antimony compound, thereby to achieve the second object of the invention.

## DESCRIPTION OF THE PREFERRED EMBODIMENTS

Iron used as the catalyst in the first method for producing 1,1,1,3,3-pentachloropropane is not limited to a particular type. Hereinafter, this catalyst will be referred to the iron catalyst, too. Examples of this iron are steels containing metallic iron, pure iron, soft iron, carbon and the like, and ferroalloys such as various stainless steels, ferrosilicon and the like. The form of iron used in the first method is not particularly limited. The iron may be in the form of powder, granule, aggregate, wire, rod, sphere, or plate. The iron having one of these forms may be arbitrarily processed into metal pieces such as packing for distillation (e.g., Raschig ring and helix), steel wool, wire netting and coil, and into other metal pieces having no particular shapes. However, it is not preferable to use a catalyst (i.e., an iron alloy) containing, in addition to iron, a large amount of component having no catalyst activity. This component may be dissolved into the reaction system or may exists as an insoluble component in the reaction system. With this, it becomes necessary to take an additional treatment of this component, after the reaction.

In the first method, it is optional to use a promoter which is at least one compound selected from Periodic Table Group 8-11 metal compounds. These compounds may be Periodic Table Group 8-11 metal complexes. The current Periodic Table Group 8-10 and 11 correspond to a former Periodic Table Group VIII and IB, respectively. Examples of the Periodic Table Group 8-11 metal compounds are metal halides, metal oxides, metal nitrides, metal acetates, and metal complexes containing acetyl acetone. Examples of metal contained in these compounds are nickel, iron, cobalt, palladium, ruthenium, copper and silver. Of the above metal compounds, metal halides selected from nickel halides, iron halides, cobalt halides, and copper halides are particularly preferable. Examples of these metal halides are metal fluorides, metal chlorides, metal bromides, and metal iodides. Of these, metal chlorides are superior to other metal halides, in view of reactivity, commercial availability, easiness of handling, and the like. Of the metal chlorides, preferable examples are ferrous chloride, ferric chloride, nickel chloride, cobalt chloride, cuprous chloride, and cupric chloride.

In the first method, the iron catalyst is preferably in an amount of at least 0,001 mol per mol of the carbon tetrachloride. It is not particularly disadvantageous to use the iron catalyst in an excessive amount, except that the reaction vessel becomes larger in the batch type reaction, because the iron catalyst exists in the form of solid in the reaction system. In the batch or half-batch type reaction, the amount of the iron catalyst is preferably from 0.001 to 1 mol, more preferably from 0.005 to 0.8 mol, and still more preferably from 0.01 to 0.5 mol, per mol of the carbon tetrachloride. It is not at all disadvantageous to use the iron catalyst in an excessive amount, in case of the continuous flow type reaction, as compared with the batch type reaction. If the iron catalyst is less than 0.001 mol, the yield may become too low.

In the first method, the promoter is preferably in an amount from 0.001 to 1 mol, more preferably from 0.005 to 0.5 mol, and still more preferably from 0.01 to 0.1 mol, per mol of the carbon tetrachloride. If it is less than 0.001 mol, the reaction rate may become loo low. The ratio of the promoter to the iron catalyst is not particularly limited and preferably not higher than 0.1 by mol.

In the first method, examples of the aprotic polar organic solvent are nitriles, amides, and other solvents much as dimethylsulfoxide, N-methyl-2-pyrrolidone, 1,3-dimethyl-2-imidazorizinone, and $\gamma$-butyrolactone. Examples of the nitriles are acetonitrile, propionitrile, butyronitrile, valeronitrile, benzonitrile, isophthalonitrile, 2-pentenenitrile, and 3-pentenenitrile. Examples of the amides are dimethylformamide. dimethylacetoamide, and hexamethylphosphoric triamide. Of these examples of the aprotic polar organic solvent, acetonitrile, dimethylformamide, hexamethylphosphoric triamide and dimethylacetoamide are particularly preferable, because metal compounds are well dissolved in these solvents.

In the first method, it is optional to add an inert solvent to the reaction system to improve reactivity and selectivity. In general, there is an advantage that the generation of a polymer of vinyl chloride is suppressed by adding such inert solvent. The amount of the inert solvent is not particularly limited. The inert solvent is not limited to a particular type, as long as it is inert in the reaction and does not act as radical scavenger.

In the first method, the molar ratio of the aprotic polar organic solvent to carbon tetrachloride is preferably from 1/10 to 10/1, more preferably from 1/2 to 2/1, and particularly preferably about 1/1. Even if this ratio is greater than 10/1, there is caused no disadvantage, except that the reaction vessel becomes larger in size. If the ratio is less than 1/10, conversion of carbon tetrachloride or of vinyl chloride becomes too small.

In the first method, the amount of vinyl chloride is not particularly limited. The amount of vinyl chloride is preferably not more than art amount equimolar with carbon tetrachloride. If it is more than the equimolar amount, the generation of a polymer of vinyl chloride may increase too much, and the amount of vinyl chloride which does not react with carbon tetrachloride may also increase too much. If the amount of vinyl chloride is less than the equimolar amount, carbon tetrachloride partly remains in the reaction vessel after the reaction. However, this remained carbon tetrachloride can be recovered by a known method such as distillation, and then reused.

In the first method, it is optional to dilute vinyl chloride with an inert gas. The degree of dilution is not particularly limited. However, as the dilution is increased, the generation of a polymer of vinyl chloride decreases. The volume ratio of the inert gas to vinyl chloride is preferably from about 0.1 to about 10, in view of the reaction efficiency. The inert gas is not limited to a particular type, as long as it is inert in the reaction and does not act as a radical scavenger. Examples of the inert gas are nitrogen gas, hydrogen gas, argon gas, and helium gas.

In the first method, the reaction temperature depends on the amounts of catalyst, of promoter, and of aprotic polar organic solvent. The reaction temperature influences conversion of vinyl chloride, selectivity of 1,1,1,3,3-pentachloropropane, and the life times of catalyst and of promoter. The reaction temperature is preferably from 80 to 150 °C and more preferably from 100 to 120 °C. If it is lower than 80 °C, the conversion of vinyl chloride may become too small. If it is higher than 150 °C, it becomes necessary to increase the inside pressure of reaction vessel during the reaction. The inside pressure of the reaction vessel, which is the total of the partial pressure of carbon tetrachloride and that of aprotic polar organic solvent at a certain temperature, is preferably from 1 to 50 kg/cm$^2$ and more preferably from 3 to 15 kg/cm$^2$.

The first method is conducted in either batch-type manner, half continuous-now type manner or continuous-flow type manner. For example, the reaction is preferably conducted at first by mixing carbon tetrachloride with aprotic polar organic solvent to prepare a mixed solution, then by adding the iron catalyst and the optional promoter to the mixed solution, and then by continuously or intermittently introducing vinyl chloride in the form of gas or liquid, into the mixed solution, thereby to react carbon tetrachloride with vinyl chloride. It is not preferable to take a first manner in which the reaction vessel is charged with the total amounts of catalyst, solvent and raw materials at the beginning of the reaction in the batch-type reaction, nor to take a second manner in which vinyl chloride in the form of liquid is gradually added. With these first and second manners, the generation of a polymer of vinyl chloride may increase too much. During the reaction, it is optional that, if the size of each particle of the iron catalyst is small (e.g., if the iron catalyst is in the form of powder), the iron catalyst is stirred together with the reaction liquid, and that the iron catalyst is fixed at a certain position and only other substances are stirred together with the reaction liquid. Even though the reaction is conducted in any manner, it becomes important to take a sufficient contact between gas and liquid. Therefore, it is preferable to use a known device or equipment to promote a sufficient contact therebetween. Examples of such device are various conventional devices such as stirrer and sparger.

In the first method, the reaction is conducted in one of first and second reactors. The first reactor is made of a material which is glass or resin, and the second reactor is lined with a material which is glass or resin. Such resin is not particularly limited, as long as it is inert in the reaction. As to such resin, it is preferable to use fluororesin. Such fluororesin is not particularly limited, and its examples are polytetrafluoroethylene, polychlorotrifluoroethylene, polyvinylidene fluoride, polyperfluoroalkylvinyl ether, polyhexafluoropropylene, copolymers of tetrafluoroethylene and perfluoroalkylvinyl ether, copolymers of tetrafluoroethylene and ethylene, and copolymers of hexafluoroethylene and tetrafluoroethylene.

After the reaction of the first method, 1,1,1,3,3-pentachloropropane having high purity can be obtained by removing the catalyst, the optional promoter, the solvent(s), the unreacted materials and the like, from crude 1,1,1,3,3-pentachloropropane, and then by conducting the rectification thereof.

In the second method, 1,1,1,3,3-pentachloropropane as a raw material may be prepared by the above-mentioned first method or by other conventional methods.

In the second method, it is assumed that the antimony catalyst takes the form of an antimony pentahalide, $SbF_aX_b$, where Sb is pentavalent, X is halogen atom, each of a and b is a number from 0 to 5, and $a + b = 5$, in the activated condition of the antimony catalyst in the reaction of the second method, regardless of the initial form of the antimony catalyst. Trivalent antimony in an antimony halide, which antimony is in the inactivated condition, is easily oxidized by chlorine, bromine, iodine or fluorine in the reaction system, to pentavalent antimony which is in the activated condition. Therefore, not only pentavalent antimony, but also antimony having a valence other than +5, may be introduced as the catalyst into the reaction system. Thus, antimony used as the catalyst in the second method is an antimony tri- or pentahalide, or metallic (elemental) antimony, or mixtures thereof. Examples of the antimony tri- and pentahalides are antimony trichloride, antimony tribromide, antimony triiodide, and antimony trifluoride, antimony pentachloride, antimony pentabromide, antimony pentaiodide, and antimony pentafluoride. Of these, antimony pentachloride and antimony trichloride are particularly preferred.

In the second method, the catalyst concentration is preferably from 0.1 to 50 mol% and more preferably from 10 to 20 mol% based on the total mol number of 1,1,1,3,3-pentachloropropane. If it is less than 0.1 mol%, both of the conversion of 1,1,1,3,3-pentachloropropane and the yield of 1,1,1,3,3-pentafluoropropane may become too small. If it is more than 50 mol%, the generation of tar composed of high boiling point compounds may increase too much and thus the catalyst may considerably deteriorate.

In the second method, the reaction temperature is preferably from 10 to 150 °C and more preferably from 50 to 130 °C. If it is lower than 10 °C, both of the conversion of 1,1,1,3,3-pentachloropropane and the yield of 1,1,1,3,3-pentafluoropropane may decrease too much. If it is higher than 150 °C, the generation of tar may increase too much and thus the catalyst may considerably deteriorate.

In the second method, the molar ratio of hydrogen fluoride to 1,1,1,3,3-pentachloropropane is preferably from 5 to 30 and more preferably from 10 to 20. If it is less than 5, the conversion of 1,1,1,3,3-pentachloropropane may be too low. If it is more than 30, the conversion of 1,1,1,3,3-pentachloropropane does not increase any more and it is not economical in view of the recovery of unreacted hydrogen fluoride.

In the second method, the reaction pressure may vary according to the reaction temperature and is not particularly limited, as long as the reaction mixture in the reaction vessel is maintained in a liquid condition. The reaction pressure is preferably from 1.0 to 100 $kg/cm^2$ and more preferably from 5 to 30 $kg/cm^2$.

In the second method, it is optional to add a solvent to the reaction system for the purpose of adjusting the reaction and of suppressing deterioration of the catalyst. Examples of this solvent are 1,1,1,3,3-pentafluoropropane, which is the aimed product, and polychlorinated compounds such as tetrachloroethane, which will hardly be chlorinated.

In the second method, when the catalyst becomes deteriorated, or the catalyst contains antimony having a valence other than +5, antimony contained in the catalyst can be easily activated into the pentavalent activated condition. This activation is conducted by introducing chlorine at a temperature from 10 to 100 $^{\circ}$C into the reaction system, in the presence of 1,1,1,3,3-pentachloropropane, 1,1,1,3,3-pentafluoropropane, or the above-mentioned optional solvent. It is optional to conduct stirring during the activation. The amount of chlorine for the activation is from 1 to 100 mols per mol of the catalyst. If the activation temperature is lower than 10 $^{\circ}$C, it takes a long time for completing the activation. If it is higher than 100 $^{\circ}$C, the coexisting 1,1,1,3,3-pentachloropropane, 1,1,1,3,3-pentafluoropropane or the optional solvent may be chlorinated.

The second method may be conducted either by the batch-type, by the half batch-type in which only the product is removed from the reaction vessel during the reaction, or by the continuous flow type. Even if either of these types is selected, it is optional to adjust the reaction condition according to need.

In the second method, it is preferable that the reaction vessel is made of a first material such as Hastelloy, stainless steel, Monel or nickel, or that the reaction vessel is lined at its inside surface with the first material, tetrafluoroethylene resin, chlorotrifluoroethylene resin, vinylidene fluoride resin, or PFA resin.

In the second method, the obtained 1,1,1,3,3-pentafluoropropane is purified by a known method regarding fluorinated compounds. For example, 1,1,1,3,3-pentafluoropropane having high purity is obtained at first by taking out the obtained 1,1,1,3,3-pentafluoropropane in the form of liquid or gas, together with hydrogen chloride and unreacted hydrogen fluoride, from the reaction vessel, then by removing hydrogen fluoride by the liquid phase separation or the like, then by removing an acid component(s) by water or a basic aqueous solution, and then by conducting distillation.

The present invention is illustrated by reference to the following nonlimitative Examples . Percentages in the following gas chromatographic analyses are by weight unless otherwise indicated. The following Examples 1-8 are illustrative of the first method of producing 1,1,1,3,3-pentachloropropane in accordance with the invention.

EXAMPLE 1

At first, 3.0 mol of carbon tetrachloride, 3.0 mol of acetonitrile, and three iron-plates were introduced into a 1-liter autoclave which is made of glass and equipped with a stirrer. Each iron plate had dimensions of 2.0 mm, 25 mm and 50 mm and weighed 20 g. As each iron-plate, JIS. G. 3141 (SPCC-SB) made by Nihon Test Panel Kogyo Co. was used, Then, the atmosphere of the autoclave was replaced by nitrogen gas. Then, the temperature of the autoclave was increased to 110 $^{\circ}$C by heating and then maintained at 110 $^{\circ}$C for 30 min, while the autoclave was sealingly closed and the mixture was stirred at a rate of 300 rpm. The pressure of the atmosphere in the autoclave was 2.5 $kg/cm^2$ at the end of the temperature maintenance for 30 min. Then, 30 min after, vinyl chloride was introduced with pressure into the autoclave so as to increase the pressure of the autoclave from 2.5 $kg/cm^2$ to 6 $kg/cm^2$. Then, vinyl chloride was gradually added into the autoclave so as to maintain the pressure at 6 $kg/cm^2$ during the reaction. Vinyl chloride added into the autoclave until the end of the reaction was in amount of 3.2 mols in total. Then, 30 hr after the start of the vinyl chloride introduction, the heating was stopped to terminate the reaction.

After the reaction, the autoclave was cooled down to room temperature. Then, the autoclave was opened to take out the reaction mixture. Then, acetonitrile and unreacted carbon tetrachloride were distilled off under reduced pressure. A remained solid matter was separated by filtration to obtain 485 g of a liquid. By a gas chromatographic analysis, it was found that this liquid contains 70.1% of 1,1,1,3,3-pentachloropropane, 16.4% of 1,1,1,3,5,5-hexachloropentane, 13.0% of 1,1,3,3,5,5-hexachlolopentane, and 0.5% of carbon tetrachloride. The yield of 1,1,1,3,3-pentachloropropane was 49.1% based on the amount of the charged vinyl chloride.

EXAMPLE 2

At first, 3.0 mol of carbon tetrachloride, 3.0 mol of acetonitrile, 0.06 mol of cuprous chloride, and three iron-plates which are the same as those in Example 1 were introduced into a 1-liter autoclave which is the same as that in Example 1. Then, the atmosphere of the autoclave was replaced by nitrogen gas. Then, the temperature of the mixture in the autoclave was increased to 100 $^{\circ}$C by heating and then maintained at 100 $^{\circ}$C for 30 min, while the autoclave was sealingly closed and the mixture was stirred at a rate of 300 rpm. The pressure of the atmosphere in the autoclave was 2.5 kg/cm$^2$ at the end of the temperature maintenance for 30 min. Then, 30 min after, vinyl chloride was introduced with pressure into the autoclave so as to increase the pressure of the autoclave from 2.5 kg/cm$^2$ to 3 kg/cm$^2$. Then, vinyl chloride was gradually added into the autoclave so as to maintain the pressure at 3 kg/cm$^2$ during the reaction. Vinyl chloride added into the autoclave until the end of the reaction was in an amount of 2.4 mols in total. Then, 8 hr after the start of the vinyl chloride introduction, the heating was stopped to terminate the reaction.

After the reaction, the autoclave was cooled down to room temperature. Then, the autoclave was opened to take out the reaction mixture. Then, acetonitrile and unreacted carbon tetrachloride were distilled off under reduced pressure. A remained solid matter was separated by filtration to obtain 428 g of a liquid. By a gas chromatographic analysis, it was found that this liquid contains 96.6% of 1,1,1,3,3-pentachloropropane, 1.6% of 1,1,1,3,5,5-hexachloropentane, 1.5% of 1,1,3,3,5,5-hexachloropentane, and 0.3% of carbon tetrachloride. The yield of 1,1,1,3,3-pentachloropropane was 79.6% based on the amount of the charged vinyl chloride.

## EXAMPLE 3

In this example, Example 2 was repeated except in that 0.06 mol of cuprous chloride was replaced by 0.03 mol of nickel chloride. The reaction time (i.e., the time from the vinyl chloride introduction start to the heating termination) was 5 hr. By the reaction, 475.1 g of a liquid was recovered. It was found that this liquid contains 95.2% of 1,1,1,3,3-pentachloropropane, 1.3% of 1,1,1,3,5,5-hexachloropentane, 3.4% of 1,1,3,3,5,5-hexachloropentane, and 0.1% of carbon tetrachloride. The yield of 1,1,1,3,3-pentachloropropane was 87.0% based on the amount of the charged vinyl chloride.

## EXAMPLE 4

In this example, Example 2 was repeated except in that 0.06 mol of cuprous chloride was replaced by 0.03 mol of ferrous chloride. The reaction time was 4 hr. By the reaction, 468.5 g of a liquid was recovered. It was found that this liquid contains 94.5% of 1,1,1,3,3-pentachloropropane, 3.9% of 1,1,1,3,5,5-hexachloropentane, 1.5% of 1,1,3,3,5,5-hexachloropentane, and 0.1% of carbon tetrachloride. The yield of 1,1,1,3,3-pentachloropropane was 85.2% based on the amount of the charged vinyl chloride.

## EXAMPLE 5

In this example, Example 2 was repeated except in that 0.06 mol of cuprous chloride was replaced by 0.06 mol of palladium chloride. The reaction time was 7 hr. By the reaction, 452.5 g of a liquid was recovered. It was found that this liquid contains 85.6% of 1,1,1,3,3-pentachloropropane, 7.2% of 1,1,1,3,5,5-hexachloropentane, 5.0% of 1,1,3,3,5,5-hexachloropentane, and 1.2% of carbon tetrachloride. The yield of 1,1,1,3,3-pentachloropropane was 74.5% based on the amount of the charged vinyl chloride.

## EXAMPLE 6

In this example, Example 2 was slightly modified as follows. That is, 3.0 mol of acetonitrile was replaced by 3.0 mol of dimethyl acetoamide. The reaction time was 6 hr. Furthermore, 200 ml of water was added to the reaction mixture taken out from the autoclave. Then, this mixture was intensely shaken, followed by the removal of the water phase. With this, 436.4 g of an organic solvent phase was recovered. By a gas chromatographic analysis, it was found that this organic solvent phase contains 98.1% of 1,1,1,3,3-pentachloropropane, 0.5% of 1,1,1,3,5,5-hexachloropentane, 0.1% of 1,1,3,3,5,5-hexachloropentane, and 0.2% of carbon tetrachloride. The yield of 1,1,1,3,3-pentachloropropane was 82.4% based on the amount of the charged carbon tetrachloride.

## EXAMPLE 7

In this example, Example 2 was repeated except in that 3.0 mol of acetonitrile was replaced by 3.0 mol of hexamethylphosphoric triamide. The reaction time was 7 hr. By the reaction, 435.3 g of a liquid was recovered. It was found that this liquid contains 90.6% of 1,1,1,3,3-pentachloropropane, 1.5% of 1,1,1,3,5,5-hexachloropentane, 7.7% of 1,1,3,3,5,5-hexachloropentane, and 0.2% of carbon tetrachloride. The yield of 1,1,1,3,3-pentachloropropane was 75.9% based on the amount of the charged vinyl chloride.

## EXAMPLE 8

At first, 2.0 mol of carbon tetrachloride, 2.0 mol of acetonitrile, 3.5 g (0.06 mol) of an iron plate, and 0.03 mol of ferric chloride were introduced into a 1-liter autoclave which is made of glass and equipped with a stirrer. This iron plate having a trade name of JIS. G. 3141 (SPCC-SB) of Nihon Test Panel Kogyo Co. had dimensions of 2.0 mm, 25 mm and 25 mm. Then, the atmosphere of the autoclave was replaced by nitrogen gas. Then, while the autoclave was sealingly closed and the mixture was stared at a rate of 300 rpm, the temperature of the autoclave was increased to 100 $^{\circ}$C by heating. Upon this, The pressure of the atmosphere in the autoclave was 2.1 kg/cm$^2$. Then, vinyl chloride was introduced into the autoclave for 2.5 hr, while the pressure of vinyl chloride was maintained at a pressure not lower than 3 kg/cm$^2$. During the reaction, the reaction temperature increased to 110 $^{\circ}$C, and the pressure increased from 2.1 kg/cm$^2$ to 2.4 kg/cm$^2$.

After the reaction, acetonitrile and unreacted carbon tetrachloride ware distilled off from the reaction vessel under reduced pressure. After the autoclave was cooled down to room temperature, a remained solid matter was separated by filtration, thereby to obtain 281.6 g of a liquid. By a gas chromatographic analysis, it was found that this liquid contains 82.3% of 1,1,1,3,3-pentachloropropane, 4.7% of 1,1,1,3,5,5-hexachloropentane, 2.8% of 1,1,1,3,5,5-hexachloropentane, and 1.1% of carbon tetrachloride. The yield of 1,1,1,3,3-pentachloropropane was 67.0% based on the amount of the charged vinyl chloride. The crude 1,1,1,3,3-pentachloropropane was purified by distillation under reduced pressure.

COMPARATIVE EXAMPLE 1

In this comparative example, Example 1 was repeated except in that the three iron-plates were replaced by 0.3 mol of ferrous chloride, that vinyl chloride introduced into the autoclave until the end of the reaction was in an amount of 0.72 mol in total, and that 6 hr after the start of the vinyl chloride injection, the heating was stopped to terminate the reaction.

After the reaction, 45.0 g of a liquid was obtained. By a gas chromatographic analysis, it was found that this liquid contains 93.4% of 1,1,1,3,3-pentachloropropane, 5.3% of 1,1,1,3,5,5-hexachloropentane, 0.8% of 1,1,3,3,5,5-hexachloropentane, and 0.1% of carbon tetrachloride. The yield of 1,1,1,3,3-pentachloropropane was 27.1% based on the amount of the charged vinyl chloride.

The following Examples 9-14 are illustrative of the method of producing 1,1,1,3,3-pentafluoropropane in accordance with the invention.

EXAMPLE 9

At first, 0.2 mol (60 g) of antimony pentachloride, 10 mol (200 g) of hydrogen fluoride, and 1 mol (216.5 g) of 1,1,1,3,3-pentachloropropane were introduced into a 1-liter autoclave which is equipped with a condenser and a stirrer and made of stainless steel (SUS 316L). Then, the reaction temperature was increased to 100 $^{\circ}$C while the reaction mixture was stirred. As the reaction proceeded, the inside pressure of the autoclave increased. When the pressure increased to 15 kg/cm$^2$, it was started to remove hydrogen chloride from the autoclave through the condenser so as to maintain the inside pressure at 15 kg/cm$^2$.

Then, 3 hr after the start of the reaction, the reaction vessel was cooled down to room temperature, and the pressure was lowered to the atmospheric pressure. A gas flown from the reaction vessel was passed through a water layer and then collected in a trap cooled by a Dry Ice-methanol mixture. The condensate and the residue in the autoclave were washed with hydrochloric acid and then with water, thereby to obtain 121.8 g of an organic matter. As a gas chromatographic analysis, it was found that this organic matter has a chemical composition as shown in Table. The results of the following Examples 10-14 are also shown in Table.

EXAMPLE 10

In this example, Example 9 was repeated except in that the reaction temperature was changed from 100 $^{\circ}$C to 75 $^{\circ}$C. With this, 129.9 g of an organic matter was obtained as the reaction product.

EXAMPLE 11

In this example, Example 10 was repeated except in that 15 mol (300 g) of hydrogen fluoride was used in place of 10 mol of the same. With this, 125.8 g of an organic matter was obtained as the reaction product.

EXAMPLE 12

In this example, Example 10 was repeated except in that 0.3 mol (90 g) of antimony pentachloride was used in place of 0.2 mol of the same. With this, 128.9 g of an organic matter was obtained as the reaction product.

EXAMPLE 13

At first, 0.05 mol (15 g) of antimony pentachloride, 7,5 mol (150 g) of hydrogen fluoride, and 1 mol (216.5 g) of 1,1,1,3,3-pentachloropropane were introduced into a 1-liter autoclave which is equipped with a condenser, a pressure regulating valve, and a stirrer and made of stainless steel (SUS 316L). Then, the reaction temperature was increased from 13 °C to 70 °C while the reaction mixture was stirred. As the reaction proceeded, the inside pressure of the autoclave increased by the generation of hydrogen chloride. When the pressure increased to 8 kg/cm$^2$, it was started to remove hydrogen chloride from the autoclave by adjusting the pressure regulating valve so as to maintain the inside pressure at 8 kg/cm$^2$.

Then, 5 hr after the start of the reaction, the reaction vessel was cooled down to room temperature, and a gas was removed from the autoclave by lowering the inside pressure the atmospheric pressure. This gas was passed through a water layer and a sulfuric acid layer and then collected in a trap cooled by a Dry Ice-methanol mixture. The thus collected condensate and the residue in the autoclave were washed with hydrochloric acid and then with water, thereby to obtain 113.1 g of an organic matter. By a gas chromatographic analysis, it was found that the conversion of 1,1,1,3,3-pentachloropropane to 1,1,1,3,3-pentafluoropropane was 100% and that this organic matter has a chemical composition as shown in Table. Besides components shown in Table, this organic matter contained 6.4% of a high boiling point matter having a carbon atom number of 6.

EXAMPLE 14

At first, 1.92 mol (576 g) of antimony pentachloride and 179 mol (3,580 g) of hydrogen fluoride were introduced into a 20-liter autoclave which is equipped with a condenser, a pressure regulating valve on the back thereof, and a stirrer and made of stainless steel (SUS 316). Then, the reaction temperature was increased to 60 °C while the reaction mixture was stirred. Upon this, it was started to continuously introduce 24 mol (5,196 g) of 1,1,1,3,3-pentachloropropane into the autoclave for 3 hr at a rate of 8 mol/hr. As the reaction proceeded, the inside pressure of the autoclave increased by the generation of hydrogen chloride. When the pressure increased to 8 kg/cm$^2$, it was started to remove hydrogen chloride from the autoclave by adjusting the pressure regulating valve so as to maintain the inside pressure at 8 kg/cm$^2$.

Then, 3 hr after the start of the reaction, the introduction of 1,1,1,3,3-pentachloropropane was stopped. After that the reaction was further continued for another 4 hr. A gas flown from the autoclave through the condenser in the middle of the reaction, and another gas flown therefrom by lowering the pressure of the autoclave to the atmospheric pressure, after the end of the reaction, were passed through a water layer and a sulfuric acid layer, and then were collected in a trap cooled by a Dry Ice-methanol mixture. The thus collected condensate derived from these gases and the reaction mixture in the autoclave were washed with hydrochloric acid and then with water, thereby to obtain 2948.1 g of an organic matter. By a gas chromatographic analysis, it was found that the conversion of 1,1,1,3,3-pentachloropropane was 100% and that this organic matter has a chemical composition as shown in Table. Besides components shown in Table, this organic matter contained 0.8 wt% of a high boiling point matter having a carbon atom number of 6.

Table

| | Reaction Product Composition (wt%) | | | |
|---|---|---|---|---|
| | HFC-245 [1] | HFC-1233 [2] | HCFC-244 [3] | HCFC-243 [4] |
| Example 9 | 69.1 | 10.7 | 3.2 | 2.2 |
| Example 10 | 45.6 | 12.2 | 14.1 | 24.2 |
| Example 11 | 50.8 | 8.3 | 15.4 | 9.4 |
| Example 12 | 45.8 | 11.5 | 11.6 | 19.9 |
| Example 13 | 77.8 | 0.4 | 13.6 | 1.8 |
| Example 14 | 97.9 | 0.5 | 0.7 | 0.1 |

1) HFC-245: 1,1,1,3,3-pentachloropropane
2) HFC-1233: 3,3,3-trifluoro-1-chloro-1-propene
3) HCFC-244: 1,1,1,3-tetrafluoro-3-chloropropane
4) HCFC-243:1,1,1-trifluoro-3,3-dichloropropane

**Claims**

1. A method of producing 1,1,1,3,3-pentachloropropane, comprising a step of reacting carbon tetrachloride with vinyl chloride in an aprotic polar organic solvent, in the presence of a catalyst comprising elemental iron.

2. A method according to Claim 1, wherein the step is conducted in the presence of said catalyst, said solvent, and a promoter which is at least one compound selected from Periodic Table Group 8-11 metal compounds.

3. A method according to Claim 2, wherein said Periodic Table Group 8-11 metal compounds are selected from the group consisting of metal halides, metal oxides, metal nitrides, metal acetates, and metal complexes containing acetyl acetone.

4. A method according to Claim 3, wherein said metal halides are selected from the group consisting of nickel halides, iron halides, cobalt halides, and copper halides.

5. A method according to Claim 4, wherein said metal halides are metal chlorides.

6. A method according to Claim 5, wherein said metal chlorides are selected from the group consisting of ferrous chloride, ferric chloride, nickel chloride, cobalt chloride, cuprous chloride, and cupric chloride.

7. A method according to Claim 1, wherein said solvent is at least one compound selected from the group consisting of nitriles, amides, dimethylsulfoxide, N-methyl-2-pyrrolidone, 1,3-dimethyl-2-imidazorizinone, and $\gamma$-butyrolactone.

8. A method according to Claim 1, wherein said solvent is at least one compound selected from the group consisting of acetonitrile, dimethylformamide, hexamethylphosphoric triamide and dimethylacetoamide.

9. A method according to Claim 1, wherein the step is conducted at first by introducing said catalyst into a liquid mixture of said carbon tetrachloride and said solvent to prepare a first mixture, and then by introducing said vinyl chloride into said first mixture, thereby to react said carbon tetrachloride with said vinyl chloride.

10. A method according to Claim 1, wherein the step is conducted in one of first and second reactors, said first reactor being made of a material which is one of glass and fluororesin, said second reactor being lined with a material which is one of glass and fluororesin.

11. A method according to Claim 1, wherein said catalyst comprises an alloy containing elemental iron.

12. A method according to Claim 1, wherein said catalyst is in an amount or at least 0.001 mol per mol of said carbon tetrachloride.

13. A method according to Claim 2, wherein said promoter is in an amount from 0.001 to 1 mol per mol of said carbon tetrachloride.

14. A method according to Claim 1, wherein the molar ratio of said solvent to said carbon tetrachloride is at least 1/10.

15. A method according to Claim 1, wherein the molar ratio of said vinyl chloride to said carbon tetrachloride is not greater than 1/1.

16. A method according to Claim 1, wherein the step is conducted at a temperature from 80 to 150 $^{\circ}$C under a pressure from 1 to 50 kg/cm$^2$.

17. A method of producing 1,1,1,3,3-pentafluoropropane, comprising a step of fluorinating 1,1,1,3,3-pentachloropropane with hydrogen fluoride in a liquid phase, in the presence of a catalyst comprising at least one of metallic antimony and an antimony compound.

18. A method according to Claim 17, wherein said antimony compound is one of an antimony pentahalide and an antimony trihalide.

19. A method according to Claim 18, wherein said antimony pentahalide is one selected from the group consisting of antimony pentachloride, antimony pentabromide, antimony pentaiodide, and antimony pentafluoride.

**20.** A method according to Claim 18, wherein said antimony trihalide is one selected from the group consisting of antimony trichloride, antimony tribromide, antimony triiodide, and antimony trifluoride.

**21.** A method according to Claim 17, wherein the step is conducted at a temperature from 10 to 150 $^{\circ}$C under a pressure from 1.0 to 100.0 kg/cm$^2$.

**22.** A method according to Claim 17, wherein the molar ratio of said catalyst to said 1,1,1,3,3-pentachloropropane is from 0.1:100 to 50:100.

**23.** A method according to Claim 17, wherein the molar ratio of said hydrogen fluoride to said 1,1,1,3,3-pentachloropropane is from 5 to 30.

**24.** A method of producing 1,1,1,3,3-pentafluoropropane, comprising the steps of:

(a) reacting carbon tetrachloride with vinyl chloride in an aprotic polar organic solvent, in the presence of a catalyst comprising elemental iron, to produce 1,1,1,3,3-pentachloropropane; and
(b) fluorinating said 1,1,1,3,3-pentachloropropane with hydrogen fluoride in an aprotic polar organic solvent, in the presence of a catalyst comprising elemental iron, to produce 1,1,1,3,3-pentafluoropropane.

| | European Patent Office | EUROPEAN SEARCH REPORT | Application Number EP 96 10 3220 |
| --- | --- | --- | --- |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
| --- | --- | --- | --- |
| A | GB-A-1 007 542 (IMPERIAL CHEMICAL INDUSTRIES LIMITED) * the whole document * --- | 1 | C07C17/278 C07C17/20 C07C19/08 C07C19/01 |
| A | FR-A-2 120 337 (RHONE-PROGIL) * claims; example 3 * --- | 1,7,8 | |
| A | EP-A-0 522 639 (SOLVAY) * claims * --- | 17-19 | |
| A | WO-A-95 04021 (ALLIED SIGNAL INC) * page 6 - page 7; example 3 * --- | 24 | |
| P,X | WO-A-96 01797 (ALLIED SIGNAL INC) * the whole document * --- | 17-19,24 | |
| E | EP-A-0 703 205 (ATOCHEM ELF SA) * the whole document * ----- | 17-19 | |
| | | | TECHNICAL FIELDS SEARCHED (Int.Cl.6) C07C |

The present search report has been drawn up for all claims

| Place of search THE HAGUE | Date of completion of the search 7 June 1996 | Examiner Bonnevalle, E |
| --- | --- | --- |

EPO FORM 1503 03.82 (P04C01)